# EUROPEAN PATENT SPECIFICATION

(11) **EP 4 212 106 B1**
(45) Date of publication and mention of the grant of the patent: **12.11.2025**
(21) Application number: 22382015.0
(22) Date of filing: 13.01.2022
(51) Int. Cl.: A61B 17/00, B05B 7/00

(54) **HANDHELD DEVICE FOR APPLYING A CYANOACRYLATE ADHESIVE COMPOSITION**
HANDGERÄT ZUM AUFTRAGEN EINER CYANOACRYLATKLEBSTOFFZUSAMMENSETZUNG
DISPOSITIF PORTABLE PERMETTANT D'APPLIQUER UNE COMPOSITION ADHÉSIVE DE CYANOACRYLATE

(43) Date of publication of application: 19.07.2023
(73) Proprietor: Cuantum Medical Cosmetics, S.L., 08193 Cerdanyola del Vallès (Barcelona) (ES)
(72) Inventor: AGUILERA CORROCHANO, Jordi, 08208 Sabadell (Barcelona) (ES); ORTEGO HUGUET, Sara, 07712 Maó (Illes Balears) (ES); SÁEZ FRAILE, Rubén, 08021 Barcelona (ES); CLOSA FERRER, Guillem, 08172 Sant Cugat del Vallès (Barcelona) (ES); SANTIAGO RANDO, Irene, 19005 Guadalajara (ES); CORREA ÁLVAREZ, Adán, 18610 Lobres (Granada) (ES); PUJOL NOGUERA, Ferran, 08635 Sant Esteve Sesrovires (Barcelona) (ES)
(74) Representative: Araujo, Daniel

(56) References cited:
- WO-A1-2018/191802
- US-A- 3 508 710
- US-A- 6 121 341
- US-A1- 2019 099 170
- US-B1- 6 439 789

## Description

### Field of the invention

The present invention relates to the field of cyanoacrylate adhesive compositions, in particular to a handheld device for applying an activated adhesive composition, which is suitable to be applied topically on skin.

### Background art

Cyanoacrylate (CA) is the generic name for a family of resistant fast acting adhesives based on esters of 2-cyanoacrylic acid. The structure of the monomer is as follows: 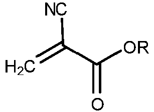 wherein R is generally an alkyl group such as, for example, methyl, ethyl, butyl or 2- octyl. Such compounds are well known for some time, as disclosed, for example, in S. Ebnesajjad Ed., Adhesives Technology Handbook, William Andrew, Norwich, 2008.

These CA compounds are ethylene monomers 1,1-disubstituted with electron withdrawing groups, so that they are highly reactive towards nucleophiles for its electron deficiency. Their structure allows an extremely easy polymerisation thereof initiated by any nucleophilic or basic species, which is inherently present in the medium or substrate to be bonded.

Said CA adhesives are widely used in industrial, consumer and biomedical applications. In the latter regard *n*-butyl-, *n*-hexyl- and 2-octyl cyanoacrylate monomers are the major components of commercial topical skin adhesives (TSAs) sold under various Brand Names, a non-exhaustive list of which includes Histoacryl^{®}, CutisSeal^{®} Topical H, and Dermabond^{®}.

All such adhesive products are applied exclusively in the form of bulk liquids from various types of handheld devices such as small plastic ampoules with fine cannulas, pen-like dispensers containing glass ampoules loaded with formulation, or even from small aluminium tubes with fine brush-tips (Exofin^{®}). Invariably the volume of formulation in such products is about 1 mL and one device is generally used to treat one or two wound sites at most on a single patient, then the device is discarded.

These types of products are used for the approximation of acute wounds for example in place of suture stitches, suture strips or staples and are approved by the United States Federal Drug Agency (FDA) as Class II Medical Devices. The adhesives should not enter the wound as they can interfere with, or delay healing, as disclosed in Bruns et al., Using tissue adhesive for wound repair: a practical guide to Dermabond, Am. Fam. Physician 2000, 61 (5), 1383-1388.

Instructions for use (IFU) accompanying this type of product clearly indicate how clean wound edges should be approximated while the adhesive is applied atop the join and along its adjacent borders. The applicator device spreads liquid adhesive that cures (polymerises) typically within one to two minutes and the wound is protected rapidly by a thus formed strong polymer film. The film lasts intact for several days after which time it sloughs off naturally leaving a healed wound with no scaring or 'train track' marks of the type associated with traditional stitching. TSAs therefore produce a good cosmetic outcome and are popular methods for wound closure especially in cosmetic surgery.

Another important use of CA TSAs is related to their application as microbial barrier for surgical incisions, commercially available under brand names such as Integuseal^{®}, including 3.5 ml of product, as disclosed in US8609128.

Aside from their use in TSA applications, CA adhesives have also been deployed in emergency situations such as in the battlefield as haemostatic agents, since components in blood and wound exudates instantly activate these materials into polymerisation on contact. In such circumstances the urgent need is to stop wounds bleeding, not to promote long term healing. CA based haemostatic agents have not been used commercially outside of military medicine since the early parts of the Vietnam War (T. Matsumoto, Tissue Adhesives in Surgery, 1972, New York, Medical Examination Publishing Company, ISBN 0-87488-756-9) and many commercial alternatives to CA exist as haemostats and have been deployed in more recent combat situations, as disclosed in, for example, Gordy et al., Military applications of novel haemostatic devices, Expert Rev. Med. Devices, 2011, 8(1), 41-47; or Trunkey et al., Lessons relearned, Arch. Surg., 2008, 143, 112-4.

In an academic study conducted by the Universities of Washington and Minnesota, a specialised fluorinated CA (Flucrylate^{®}) was examined as a haemostatic agent for the treatment of ulcers. That study used a lab-based delivery system in which the CA was admixed with a Freon^{®} propellant in a pressure bottle and a second CO₂ gas stream was admixed with a pressure-released CA stream to create a fine mist spray. The second gas stream did not activate the CA and a foot pedal was used to control the supply of CO₂ from a gas cylinder.

The application relied on the 'substrate' to cause the sprayed formulation to activate on contact with the wound site. The CA failed to adequately seal the ulcers, as disclosed in Protell et al., Failure of Cyanoacrylate Tissue Glue (Flucrylate, MBR4197) to Stop Bleeding from Experimental Canine Gastric Ulcers, Am. J. Dig. Dis., 1978, 23(10), 903-8.

The successful use of a sprayed *n*-butyl CA, but the unsuccessful use of sprayed 2-octyl CA exclusively directed to haemostatic wound treatment, is disclosed in more recent further research publications, such as, for example, Toapanta-Yanchapaxi et al., Cyanoacrylate spray as treatment in difficult-to-manage gastrointestinal bleeding, World J. Gastroinstest. Endo., 2014, 6(9), 448-452; Prachayakul *et al.,* Spraying *n*-butyl-2-cyanoacrylate (Histroacryl) as a rescue therapy for gastrointestinal malignant tumor bleeding after failed conventional therapy, Endoscopy 2011, 43, E227-8; Walia et al., Cyanoacrylate spray for treatment of difficult-to-control GI bleeding, Gastrointest. Endos., 2013, 78(3), 536-9; and Shida et al., Spraying n-butyl-2-cyanoacrylate (Histoacryl) might be a simple and final technique for bleeding gastrointestinal lesions, Endoscopy, 2009, 41, E27-8.

US patent applications US-A-2009/0246262 and US-A-2014/0228475 also refer to the application of a medical grade CA by spray. The former discloses a method for treating a puncture wound, which comprises the provision of a biocompatible alkyl cyanoacrylate composition that can polymerize upon contact with body fluid; and the introduction of said composition into a puncture wound to seal the wound and reduce bleeding. The latter discloses a conformable coating, which provides a highly durable and resilient film useful for protecting and repairing surfaces such as skin and mucous membranes, wherein said composition comprises a polymerizable cyanoacrylate monomer, an elastomer, and a volatile liquid.

European patent application EP-A-3144073 discloses a device for spraying a volatile initiator over a CA composition for activating said composition, wherein the method for activating a curable material comprises the steps of: i) applying a curable material to a substrate on which it is to be cured; and, ii) dispensing a gaseous form of an activator component onto the curable material to activate the curable material.

Another approach is the use of sprayable liquid bandages, such as Nexcare^{®}, New-Skin^{®} and Elastoplast Spray Plaster, well known consumer products for the protection of minor cuts and grazes, which comprise pre-formed polymers in volatile solvents. Said kind of products are also disclosed in, for example, US3987000 and US7641893.

US6439789 discloses polymerizable 1,1-disubstituted ethylene monomer formulation applicator, comprising: an applicator tip with an internal cavity capable of receiving a polymerizable 1,1-disubstituted ethylene monomer, the applicator tip having a female fitting that defines an open end communicating with the internal cavity, the applicator tip including a porous application surface; and a container of polymerizable 1,1-disubstituted ethylene monomer, the container having a male fitting that is engaged with the female fitting so that the container is in fluid communication with the internal cavity during use.

However, missing to date is a convenient and safe means and device for rapidly sealing deep or surgical wounds with a highly reactive monomer composition that does not rely on the nature of a wound site or undamaged skin to which it is to be applied to initiate its polymerisation but will nevertheless instantly polymerise atop such substrates to form a protective or antimicrobial film without the device directly touching the application site and without the need to manually rupture hermetically sealed glass ampoules that present risk to the professional end-user and/or patient.

Thus, there is still a need to provide a compact device for spraying and activating an adhesive composition for topical application to living skin which is further characterized by a low volume for single use yet of economic and ergonomic design.

### Subject-matter of the invention

The subject-matter of the present invention is a handheld device for applying a cyanoacrylate adhesive composition.

### Figures

Figure 1 (a) is a schematic representation of a moulded actuator for use in one embodiment of the invention illustrating two concentric internal conduits, a non-pressurised compartment with a sealable filling port and a dispensing nozzle, and a temporary plug or pin in place; and Figure 1 (b) as (a) but having temporary plug or pin removed.
Figure 2 shows a schematic representation of a handheld device for spraying an activated cyanoacrylate composition deploying an actuator having a non-pressurised compartment within.
Figure 3 (a) shows a schematic representation of a moulded actuator for use in one embodiment of the invention illustrating a disc filter of porous material (i.e., porous matrix) in place of a dispensing nozzle and a protective cover for the disc in place. Figure 3 (b) as (a) but having the protective cover for the disc removed.
Figure 4 shows a schematic representation of a handheld device for spraying an activated cyanoacrylate composition that is propelled through a porous matrix that contains an initiator composition.

The device of the invention represented schematically in the attached figures shows the following features:
- 1: Moulded actuator
- 2: Dispensing nozzle with central orifice
- 3: Non-pressurised compartment
- 4: L-shape conduit
- 5: End of L-shape conduit engaged with a valve in a pressurised canister
- 6: Stopper
- 7: Filling port
- 8: Plug
- 9: Activated spray
- 10: Disc filter of porous material (i.e., porous matrix)
- 11: Retaining ring
- 12: Protective cover
- 13: Spring-loaded valve
- 14: Pressurised canister
- 15: Outer housing
- 16: Cap
- 17: Handheld device

### Detailed description of the invention

The object of the present invention is a handheld device for applying a cyanoacrylate adhesive composition, characterized in that it comprises:
a) a pressurized canister comprising a cyanoacrylate composition comprising a cyanoacrylate monomer, and an initiator composition comprised in a non-pressurized compartment,
   wherein the cyanoacrylate composition is propelled from the pressurized cannister and the initiator composition is aspirated from the non-pressurized compartment; or
b) a pressurized canister comprising a cyanoacrylate composition comprising a cyanoacrylate monomer, and a porous matrix comprising an initiator composition entrained in the porous matrix,
   wherein the cyanoacrylate composition is propelled through the porous matrix comprising an initiator composition entrained therein; or
c) a pressurized canister comprising an initiator composition and a non-pressurized compartment comprising a cyanoacrylate composition comprising a cyanoacrylate monomer,
wherein the initiator composition is propelled from the pressurized cannister and the cyanoacrylate composition is aspirated from the non-pressurized compartment, and
wherein the confluence of the cyanoacrylate composition and of the initiator composition occurs in a common activated spray stream.

In a first embodiment the handheld device for applying a cyanoacrylate adhesive composition comprises a pressurized canister comprising a cyanoacrylate composition comprising a cyanoacrylate monomer, and an initiator composition comprised in a non-pressurized compartment,
wherein the cyanoacrylate composition is propelled from the pressurized cannister and the initiator composition is aspirated from the non-pressurized compartment, and
wherein the confluence of the cyanoacrylate composition and of the initiator composition occurs in a common activated spray stream.

In a second preferred embodiment the handheld device for applying a cyanoacrylate adhesive composition comprises a pressurized canister comprising a composition comprising a cyanoacrylate monomer, and a porous matrix comprising an initiator composition entrained therein,
wherein the cyanoacrylate composition is propelled through the porous matrix comprising an initiator composition,
wherein the confluence of the cyanoacrylate composition and of the initiator composition occurs in a common activated spray stream.

In a third embodiment the handheld device for applying a cyanoacrylate adhesive composition comprises a pressurized canister comprising an initiator composition and a non-pressurized compartment comprising a cyanoacrylate composition comprising a cyanoacrylate monomer,
wherein the initiator composition is propelled from the pressurized cannister and the cyanoacrylate composition is aspirated from the non-pressurized compartment, and
wherein the confluence of the cyanoacrylate composition and of the initiator composition occurs in a common spray stream.

The first and third embodiments of the present invention rely on the Venturi effect to aspirate one composition contained in a non-pressurised compartment, into a propelled stream of the other composition resulting in a spray of an activated cyanoacrylate formulation used for the treatment of wounds. In the first embodiment, the initiator composition is in the non-pressurised compartment. In the third embodiment the cyanoacrylate composition is in the non-pressurised compartment.

The inventors of the present invention have developed a handheld device providing convenient and safe means of rapidly sealing deep or surgical wounds using a highly reactive monomer composition that instantly polymerises to form a strong film without the device directly touching the wound and without presenting any risk to the clinician or patient by malfunction of the device in use.

In the present description, as well as in the claims, the singular forms "a", "an" and "the" include the plural reference unless the context clearly indicates otherwise. The ranges defined by the preposition the terms "between ... and ..." or by the terms "from ...to..." include also the two ends thereof. The term "about" refers to a deviation of plus/minus 10%, preferably plus/minus 5%. The percentages are expressed in % by weight (wt%), unless stated the contrary.

The term "activate" is used herein to mean to trigger or initiate the chemical polymerisation of cyanoacrylate monomers in cyanoacrylate compositions.

The term "cyanoacrylate monomer" is used herein to mean an ester comprising the cyanoacrylate functional group in the molecule.

The term "cyanoacrylate composition" is used herein to designate a composition comprising a cyanoacrylate monomer and a further component selected from free radical polymerization inhibitors, acid stabilizing agents, thickening agents, plasticizers, thixotropic agents, accelerating agents, solvents, dyestuffs, and mixtures thereof.

The term "cyanoacrylate adhesive composition" is used herein to designate a composition comprising a cyanoacrylate monomer and one or more further components, which also includes an initiator to initiate the polymerization of the cyanoacrylate monomer.

The term "substrate" is used herein to mean a human or an animal organ.

The term "living skin or living organ" is used herein to mean to refer to human or animal skin or organs.

The term "composition" is used herein to indicate a concoction of chemical compounds, and it is also used interchangeably with the term "formulation".

The term "initiator" is used herein to designate chemical compound(s) responsible for starting a polymerization reaction.

The term "confluence" is used herein to mean the conjoining of two flowing streams.

The term "propelled" is used herein to indicate forced ejection of a composition by a propellant.

The term "propellant" is used herein to designate a gas, a liquified-gas, or a mixture thereof used to create pressure within or onto a composition so as to eject it in the form of a spray.

The term "pressurised canister" is used herein to designate a container with contents in excess of 1 atmosphere pressure at room temperature.

The term "non-pressurised compartment" is used herein to designate a container being at about 1 atmosphere pressure at room temperature.

The term "porous matrix" is used herein to designate a filter, sponge or frit of open cell structure.

### Cyanoacrylate composition

The cyanoacrylate (CA) composition of the invention comprises a cyanoacrylate monomer and a further component selected from radical polymerization inhibitors, acid stabilizing agents, thickening agent, plasticizers, thixotropic agents, accelerating agents, solvents, dyestuffs, and mixtures thereof.

### Cyanoacrylate monomer

The cyanoacrylate (CA) monomer for use in the invention is selected from the group of n-butyl cyanoacrylate, n-hexyl cyanoacrylate, 2-octyl cyanoacrylate, and mixtures thereof.

At ambient room temperature, about 25 °C, CA monomers are low viscosity (typically 0.001-0.01 Pa.s or 1-10 cP.s) liquids that are readily polymerised.

Said monomers, being commercially available, may also be prepared by methods known by the skilled in the art such as the method described, for example, in US2467927.

The content of CA monomer is comprised between 70 wt% and 98 wt%, preferably between 75 wt% and 95 wt%, and more preferably between 80 wt% and 90 wt%, wherein the wt% is based on the total weight of the cyanoacrylate composition.

### Radical polymerisation inhibitor

The CA composition of the invention comprises radical polymerisation inhibitors to prevent premature polymerisation due to any radical mechanism such as autoxidation or thermal polymerisation.

Radical polymerisation inhibitors are selected from 4-methoxyphenol, hydroquinone, hydroquinone monomethyl ether, hydroxytoluene butyl ether, hydroxyanisole butyl ether, 2,2'-methylenebis(4-methyl-6-tert-butylphenol), 4,4'-methylenbis(2,6-di-*tert*-butylphenol) and mixtures thereof; preferably selected from the group consisting of hydroxyanisole butyl ether, 2,2'-methylenebis(4-methyl-6-tert-butylphenol), hydroxytoluene butyl ether, and mixtures thereof.

The content of the radical polymerisation inhibitor is comprised between 0.05 wt% and 1.5 wt%, preferably between 0.08 wt% and 0.6 wt%, and more preferably between 0.1 wt% and 0.5 wt%, wherein the wt% is based on the total weight of the cyanoacrylate composition.

Radical polymerisation inhibitors are commercially available through companies, such as, for example, TCI Chemical or Sigma-Aldrich.

### Acid stabilizing agent

In an embodiment, the CA composition further comprises an acid stabilizing agent.

Acid stabilizing agents are inhibitors of the anionic polymerisation and may be selected from the group consisting of Bronsted acids, Lewis acids, and mixtures thereof.

The acid stabilizing agent may be selected from boron trifluoride, boron trifluoride etherate, boron trifluoride dihydrate, trimethylsilyl triflate, sulphur dioxide, methanesulfonic acid, and mixtures thereof; preferably it is selected from sulphur dioxide, boron trifluoride etherate, methanesulfonic acid, and mixtures thereof.

In an embodiment, the composition further comprises a combination of boron trifluoride etherate and methanesulfonic acid.

In an embodiment, the composition further comprises a combination of sulphur dioxide and boron trifluoride etherate.

In an embodiment, the composition further comprises a combination of sulphur dioxide, boron trifluoride etherate and methanesulfonic acid.

The content of sulphur dioxide is usually comprised between 0.0005 wt% and 0.03 wt%, preferably between 0.0006 wt% and 0.025 wt%, more preferably between 0.00065 wt% and 0.02 wt%, wherein the wt% is based on the total weight of the composition.

The content of boron trifluoride etherate is usually comprised between 0 and 0.01 wt%, preferably between 0.0001 wt% and 0.008 wt%, more preferably between 0.0005 wt% and 0.005 wt%, wherein the wt% is based on the total weight of the composition.

The content of methanesulfonic acid is usually comprised between 0 and 0.005 wt%, preferably between 0.0005 wt% and 0.003 wt%, more preferably between 0.00075 wt% and 0.0015 wt%, wherein the wt% is based on the total weight of the composition.

### Thickening agent

A thickening agent may be added to increase the viscosity of the CA composition. A suitable thickener or thickening agent for the composition may be selected from those which are compatible with the CA monomers contained therein.

Polymers can be used as thickeners, such as, for example, poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, and mixtures thereof.

Certain fumed silica fillers, optionally treated with polydialkylsiloxanes or trialkoxyalkylsilanes, can also be used as thickening agent.

In the scope of the invention, the thickening agent is selected from poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, fumed silica, fumed silica treated with polydialkylsiloxanes or trialkoxyalkylsilanes, and mixtures thereof.

Preferably, in the CA composition the thickener is selected from vinyl acetate copolymers, fumed silica, and mixtures thereof.

Generally, the content of thickener is comprised between about 3 wt% and about 20 wt%, preferably between about 5 wt% and about 18 wt%, and more preferably between about 5 wt. % and about 15 wt%, wherein the wt% is based on the total weight of the CA composition.

These thickening agents are well known to the skilled person and have been described in the prior art.

Polymers suitable as thickening agent are commercially available through companies such as, for example, Wacker, Evonik or Lanxess.

Fumed silica is commercially available through companies such as, for example, Evonik.

### Plasticizer

The CA composition may comprise a plasticizer. Usually, the plasticizer is selected from organic esters such as, for example, acetates, carbonates, citrates, succinates, azelates, adipates, sebacates, stearates, myristates and phthalates.

Preferably the plasticizer is selected from triacetin, propylene carbonate, and mixtures thereof.

Typically, the content of the plasticizer in the composition is comprised between about 1 wt% and about 20 wt. %, preferably between about 3 wt% and about 15 wt. %, more preferably between about 5 wt% and about 12 wt%, and yet more preferably between about 8 wt% and about 11 wt%, wherein the wt% is based on the total weight of the CA composition.

### Thixotropic agent

A thixotropic agent may be added to the CA composition to manage its rheological behaviour.

Preferably, the thixotropic agent is silica, which is available as fumed silica, optionally hydrophobized, and precipitated silica. In a more preferred embodiment, it is hydrophobized fumed silica. Silica may act both as thickening agent and as thixotropic agent.

Typically, the content of thixotropic agent in the composition is comprised between about 1 wt% and about 10 wt%, preferably between about 2 wt% and about 8 wt%, and more preferably between about 3 wt% and about 5 wt%, wherein the wt% is based on the total weight of the CA composition.

Fumed silica is commercially available through companies such as, for example, Evonik.

### Accelerating agents

The CA composition may further comprise an accelerating agent.

A suitable accelerating agent or accelerator for the composition is a crown ether.

Typically, the content of the accelerating agent in the composition is comprised between about 0.01 wt% and about 0.8 wt%, preferably between about 0.05 wt% and about 0.5 wt%, and more preferably between about 0.1 wt% and about 0.3 wt%, wherein the wt% is based on the total weight of the CA composition.

### Solvents

The CA composition may further comprise a solvent to regulate the viscosity thereof.

The viscosity of the cyanoacrylate composition at room temperature is usually comprised between 0.001 and 0.5 Pa.s (1 and 500 cP.s) more preferably between 0.002 and 0.3 Pa.s (2 and 300 cP.s) and most preferably between 0.003 and 0.2 Pa.s (3 and 200 cP.s)

Viscosity can also be regulated in the cyanoacrylate composition by use of a solvent or solvents such as hexamethyldisiloxane (HMDS), octamethylcyclotetrasiloxane, decamethylcyclopentasiloxane, octamethyltrisiloxanes and the like, which do not initiate polymerization of cyanoacrylate monomers. Other volatile solvents, include volatile organosilicones, such as caprylyl methicone, ethyl trisiloxane, and the like; (C₆ to C₁₀) alkanes, such as isooctane, octane, nonane and decane, and their structural isomers, including cyclic isomers such as cyclohexane, methylcyclohexane and the like.

The propellant may also function as a solvent.

If a solvent is used, it is present in the range comprised between 10 wt% and 50 wt% based on the total weight of the CA composition.

### Dyestuff

The CA composition may further comprise a dyestuff to facilitate marking its presence or position on and around a wound. The colourants if used must not destabilise the CA compositions and must be soluble throughout the live time of the formulated product. Preferred dyestuffs are dyes selected from the group D&C Violet No. 2 (1-hydroxy-4-[(4-methylphenyl) amino]-9,10-anthracenedione), FD&C Blue No. 2 (2-(13-dihydro-3-oxo 5-sulfo-2H-indol-2-ylidene)-2,3-dihydro-3-oxo-1H-indole 5-sulfonic acid disodium salt), Solvent Green No. 3 (1 ,4-dip-toluidino-9,10-anthraquinone (PTA)), C.I. Solvent Green 5 (3,9-perylenedicarboxylicacid bis(2-methylpropyl)ester), D&C Green No. 6 (1-hydroxy-4-[(4-methylphenyl)amino]-9,10 anthracenedione), FD&C Red No. 3 (9-(o-carboxyphenyl) 6-hydroxy-2,4,5,7-tetraiodo-3H-xanthen-3-one, disodium salt, monohydrate), C.I. Acid Red 50 (sulforhodamine G), or C.I. Acid Red 52 (sulforhodamine B monosodium salt).

If present, the dyestuff is used in the concentration range typically comprised between 50 ppm and 500 ppm. In an embodiment, it is preferred not to include a dyestuff, for example on facial wounds.

### Initiator composition

The initiator of the present invention is selected from xanthines, quaternary ammonium salts, metal salts of carboxylic acids, amines, phosphines, and mixtures thereof.

Xanthines are compounds that belong to a chemical group denominated purine bases, which includes among others, guanine, adenine, hypoxanthine, caffeine, theobromine and theophylline. Xanthines selected from the group consisting of caffeine and theobromine are preferably used.

The quaternary ammonium salts can also be used as initiators, choline chloride is preferably used.

The carboxylic acids are preferably selected from monocarboxylic acids having C₂-C₂₀ linear or branched, saturated or unsaturated chain, preferably acetic acid, 2-ethylhexanoic acid, palmitic acid, and stearic acid; acrylic acid, and methacrylic acid. The metal salts are preferably selected from alkali salts, alkaline earth salts, aluminium salts, and zinc (II) salts, more preferably from lithium, sodium, potassium, calcium, magnesium, barium, aluminium, and zinc salts. Still more preferably, the metal salts are selected from the group consisting of lithium stearate, sodium stearate, potassium stearate, zinc stearate, calcium stearate, magnesium stearate, barium stearate, lithium 2-ethylhexanoate, sodium 2-ethylhexanoate, potassium 2-ethylhexanoate, zinc 2-ethylhexanoate, calcium 2-ethylhexanoate, magnesium 2-ethylhexanoate, barium 2-ethylhexanoate, sodium acetate, calcium acetate, zinc acetate, calcium (meth)acrylate, barium (meth)acrylate, aluminium (meth)acrylate, and zinc (meth)acrylate; more preferably is calcium stearate, barium stearate, calcium acrylate, barium acrylate. The commercial metal salts of stearic acid are generally mixtures of palmitic and stearic acids in different proportions, although the skilled in the art denominates them stearates.

The amines are selected from, for example, aniline, *o*-toluidine, *m*-toluidine, *p*-toluidine, *N-*methylaniline, *N*-ethylaniline, *N*-n-propylaniline, *N*-isopropylaniline, *N*-methyl-*o*-toluidine, *N-*methyl-*m*-toluidine, *N*-methyl-*p*-toluidine, *N*-ethyl-*o*-toluidine, *N*-ethyl-*m*-toluidine, *N*-ethyl-*p-*toluidine, *N,N*-dimethylaniline, *N,N*-diethylaniline, *N,N*-di-*n*-propylaniline, *N,N-*diisopropylaniline, *N*-methyl-*N*-ethylaniline, *N*-methyl-*N*-n*-*propylaniline, *N-*ethyl*-N-n-*propylaniline, *N,N*-dimethyl-*o*-toluidine, *N,N*-dimethyl-*m*-toluidine, *N,N*-dimethyl-p-toluidine, *N,N*-diethyl-*o*-toluidine, *N,N*-diethyl-*m*-toluidine, *N*, *N*-diethyl-*p*-toluidine and *N,N*-dimethyl-3-aminophenol.

Examples of suitable phosphines include, but are not limited to, phosphines such as aromatic-containing phosphines, for example, phenylphosphine, diphenylphosphine, and triphenyl phosphine; alkyl-containing phosphines such as methyl phosphine, diethyl phosphine, ethyl phosphine, dichlorodiphenyl phosphine, dichlorophenyl phosphine, and dimethyphenyl phosphine. The phosphines can be a salt, include a metal halogen and/or a functional group. Some examples: 2,2'-bis(diphenylphosphino-1,1'-biphenyl; 4,12-bis(diphenylphosphino)-[2.2]-paracyclopha-ne; 4,12-bis[di(3,5-xylyl)phosphino]-[2.2]-paracyclophane; and 1,4-bis(diphenylphos-phino)butane.

The preferred initiator in the present invention is selected from the group of quaternary ammonium salts and tertiary amines, such as choline chloride, benzalkonium chloride, triethanolamine and the like.

The initiator concentration depends on their potency but typically it is comprised between 100 ppm and 10.000 ppm in the cyanoacrylate adhesive composition.

Initiator compositions may be single or multiple component neat mixtures or may be in solution in organic or inorganic solvents or solvent mixtures, or in suspension in a liquid carrier (non-dissolving solvent), or entrained within an open cell porous matrix, wherein the pores withhold liquid forms or the pore structure is coated onto the pore wall architecture, as explained below.

Evaporation of organic solvents provides a cooling or anaesthetic effect. Additionally, coolant can be included within the initiator composition or may themselves be initiating species.

Preferred solvents are selected from the group, water, alcohols, and the like.

Optionally coolants may be used in the initiator composition and are selected from the group of volatile aprotic solvents, including volatile ketones such as menthone, acetone and methyl ethyl ketone, volatile ethers such as diethyl ether, ethyl propyl ether, dipropyl ether and dipropylene glycol dimethyl ether, volatile fluorocarbons, such as pentafluoropropane, perfluoroheptane, perfluoromethylcyclohexane, acetates such as methyl and ethyl acetate, propylene glycol diacetate and the like.

In an embodiment of the present invention, the initiator composition contains a propellant or propellant mixture that aids its forceful expulsion on demand when a stream of initiator is to be created. Propellants and preferred propellants are described below.

### Propellant

In the device of the present invention, either the cyanoacrylate composition or the initiator composition may be in a pressurised canister, which comprises a propellant to produce a spray.

The use of propellants in medical devices is well known for example in inhalers and nasal sprays and is described in detail by, for example, Kulkarni, V.S. and Shaw, C., Essential Chemistry for Formulators of Semisolid and Liquid Dosages, Chapter 6, 'Aerosols and Nasal Sprays', pages 71-97, Elsevier, 2016, ISBN 978-0-12-801024-2.

Propellants commonly used in pharmaceutical aerosols include both liquefied gases (chlorofluorocarbons (CFCs), hydrocarbons, hydrochlorofluorocarbons (HCFC), and hydrofluorocarbons (HFCs)) and compressed gases, (nitrogen, nitrous oxide (N₂O), and carbon dioxide).

When liquefied-gas propellants are sealed in a pressurized canister with either composition, equilibrium is established between the propellant that remains liquefied and the portion that vaporizes and occupies the headspace of the pressurized canister. The vapor pressure at equilibrium is characteristic for each propellant at a given temperature, and is independent of the quantity of liquefied phase present. When the pressurized canister is opened, for example by actuation of a spring-loaded valve, the internal high pressure forces the composition out of the canister. When the propellant reaches the air, it evaporates due to the drop in pressure and leaves the composition as airborne liquid.

When compressed gases used as propellants the gas is mostly in the headspace of the pressurized canister, and the pressure of the gas forces the composition out when the canister is opened for example by actuation of a spring-loaded valve.

Examples of CFC propellants are trichlorofluoromethane (CCl₃F, Propellant 11); dichlorodifluoromethane (CCl₂F₂; Propellant 12); and 1,2-dichloro- 1,1,2,2-tetrafluoroethane (ClF₂C-CClF₂, Propellant 114). These propellants are gases at room temperature that can be liquefied by cooling below their boiling point or by compressing at room temperature. These liquefied gases also have a very large expansion ratio compared to the compressed gases (e.g., nitrogen, carbon dioxide). Due to their role in depleting the ozone layer of the atmosphere, the use of CFC propellants is restricted to pharmaceutical aerosols used in the treatment of asthma and chronic obstructive pulmonary disease. Propellants 11, 12, and 114 are the choice for oral, nasal, and inhalation aerosols due to their relatively low toxicity and inflammability.

HCFC and HFC propellants break down in the atmosphere at a faster rate than the CFCs, resulting in a lower ozone-depleting effect. Examples of HCFC and HFC propellants are chlorodifluoromethane (CHClF₂; Propellant 22); 1,1,1,2-tetrafluoroethane (CF₃CH₂F; Propellant 134a); 1-chloro-1,1-difluoroethane (CH₃CClF₂; Propellant 142b); 1,1-difluoroethane (CH3CHF2; Propellant 152a); and 1,1,1,2,3,3,3-hepta-fluoropropane (CF₃CHFCF₃; Propellant 227). Propellants 22, 142b, and 152a are used in topical pharmaceuticals. The latter three propellants have good miscibility with water, which makes them more useful as solvents compared to some other propellants. Medicinal aerosols such as asthma inhalers use the HFC propellants 134a, 227, or a combination of both, as disclosed in US-A-2015/0250713.

Hydrocarbon propellants are used in topical pharmaceutical aerosols because of their environmental acceptance, low toxicity, and lack of reactivity. These are flammable. Propane (Propellant A-108), butane (Propellant A-17), and isobutane (Propellant A-31) are the most commonly used hydrocarbons. They are used alone, as mixtures, or mixed with other liquefied gases to obtain the desired vapor pressure, density, and degree of flammability.

Examples of compressed-gas propellants include gases such as nitrogen (N₂), nitrous oxide (N₂O), and carbon dioxide (CO₂) have been used as aerosol propellants for products that are dispensed as fine mists, foams, or semisolids-including food products, dental creams, hair preparations, and ointments.

Recently a new class of hydrofluoro olefins (HFOs) or hydrochlorofluro olefins (HCFOs) have emerged with low Global Warming impact such as (HFO-1234ze(E) (trans-1,3,3,3, tetrafluoroprop-1-ene), (HFO-136mzz) (trans-1,1,1,4,4,4-hexafluoroprop-2-butene), that have been used as non-flammable air dusters. HFOs have been described in the patent and scientific literature, for example in US-A- 2014/284516, WO-A-2015/126662, and in M. A. Garcia, Patent Picks: New Propellant Technologies, Chem. Eng. News, 2015, 93(47), 32-35. In the present invention the preferred propellants are selected from Propellant 11, 12, 114, 134a, 227, A-108, A-17, A-31, N₂, N₂O, CO₂, air, oxygen enriched compressed nitrous oxide (N₂O), oxygen enriched compressed carbon dioxide, HFO-1234ze(E), HFO-136mzz, and mixtures thereof.

The propellant used for the cyanoacrylate composition may be the same or different to that of the initiator composition.

Where water as a solvent is used in the initiator composition of the present invention Propellants 134a and 227 are preferred. Water cannot be used in a mixture of a cyanoacrylate composition however.

The concentration of the propellant relative to either composition determines the performance of the spray and its convenience of use it is typically adjusted in the range comprised between 50 wt% and 90 wt%.

### Antimicrobial agent

Either the cyanoacrylate or the initiator compositions, or both, may optionally include antimicrobial agents. Although cured cyanoacrylate films are known to provide a microbial barrier to certain common organisms known to cause surgical site infections, as disclosed in, for example, Rushbrook et al., The antibacterial effect of 2-octyl cyanoacrylate (Dermabond®) skin adhesive, J. Infect. Prev., 2014, 15(6), 236-9; Bhende et al., In Vitro Assessment of Microbial Barrier Properties of Dermabond® Topical Skin Adhesive, Surg. Infect., 2002, 3(3), 251-7; Friedman et al., 1-Hexyl-2-cyanoacrylate compound (Neucrylate) bactericidal properties, J. Neurointervent. Surg., 2012, 4, 379-384; or Quinn et al., N-2-butylcyanoacrylate: Risk of bacterial contamination with an appraisal of its antimicrobial effects, J. Emerg. Med., 1995, 13(4), 581-5, in order to reinforce antimicrobial properties, especially against gram negative bacteria and fungi, including an antimicrobial agent may be advantageous.

In an embodiment, either the cyanoacrylate or the initiator compositions, or both, include at least one antimicrobial agent.

The handheld device of the present invention provides means by which broad spectrum antimicrobial agents, that would have limited solubility or stability on a practical timescale if formulated into cyanoacrylate compositions, may still be utilised since they can be delivered in part or alone from the separated initiator composition where greater latitude for formulation prevails. Antimicrobial agents may even act as an initiator or boost the efficacy of known selected initiators. Non-limiting examples of antimicrobial agents that are compatible with cyanoacrylate monomers include polyvinylpyrrolidone-iodine, as disclosed in, for example, US5684042, and diiodomethyl-p-tolylsulfone, as disclosed in, for example, Rev. Cubana Invest. Bioméd., 2017;36(1):1-6), chitosan is an example of a useful antimicrobial agent yet has no long-term stability in cyanoacrylate formulations, as disclosed in, for example, EP-A-2200690.

### Handheld device

The device of the invention is a handheld device in an embodiment of the invention.

The device comprises a protective cap, a moulded actuator, an outer housing, and a pressurized canister comprising an actuable valve.

### Protective cap

A cap for the handheld device delivering the inventive compositions is provided to protect the actuator, maintain product hygiene and prevent inadvertent actuation. The cap may be a simple moulded part that fits atop the main housing of the device.

Typically, the protective cap is constructed of a moulded medical grade plastic such a poly (methyl methacrylate) (PMMA) and may be either transparent or opaque and coloured or not.

### Moulded actuator

The moulded actuator is usually formed from a medical grade plastic, preferably PMMA, and ergonomically shaped in the form of a depressible finger-activated button of the type common in medical devices such as asthma inhalers or spray-on bandages. The purpose of the moulded actuator is to engage with the releasable valve in the pressurized canister and convey its contents through conduit or conduits within the plastic part. Mechanical engagement may be effected by a so-called hollow stem that is either moulded as part of the actuator, alternatively the stem may be provided with the pressure canister. The engagement of actuators onto valves in pressurized canisters is well known in the prior art in the field of aerosol canisters, for example.

The moulded actuator has different designs depending on the inventive embodiment. In brief, it either comprises a non-pressurised compartment within or not:

### A) With a non-pressurised compartment

The purpose of a non-pressurised compartment is to contain one or other of the inventive liquid compositions at normal ambient atmospheric pressure. This is a compartment having a volume of approximately 0.3 - 0.8 mL, preferably of circular cross-section that forms a cylindrical, or partially cylindrical first conduit with its long axis perpendicular to the axis along which the valve on the pressurized cylinder is actuated. The cross-sectional diameter of the first conduit is comprised between 5 mm and 10 mm. In one embodiment the cylindrical conduit is terminated at one end with a dispensing nozzle, which is a separate plastic disc part of diameter comprised between 3 mm and 6 mm with a central orifice having a diameter comprised between 0.2 mm and 1 mm. An end wall of any shape closes off the other end of the first cylindrical conduit. Within the first conduit is a second conduit of smaller diameter in the form of a hollow L-shaped tube with an internal diameter comprised between 0.5 mm and 3 mm. One leg of the L-shape has the same central axis as the first conduit, the other leg of the L-shape is oriented toward the pressurized canister. One end of the second conduit terminates close to the dispensing nozzle disc within a distance comprised between 0.5 mm and 2 mm. The other end of the second conduit engages with the pressurized canister via a valve.

A removable plug or pin is inserted through the orifice of the dispensing nozzle and into the bore or the second conduit thus initially sealing both.

The compartment is accessed by a sealable port so it can be filled with an inventive composition. Once filled, the port is hermetically sealed with a press fitted stopper preferably made from the same plastic as the moulded actuator.

In different embodiments of the present invention, the non-pressurised compartment is filled with either an initiator composition, or a cyanoacrylate composition. In the former, the cyanoacrylate composition is contained in the pressurized canister, in the latter, the initiator composition is contained in the pressurized canister.

Due to the desire to limit volume for single-use these particular embodiments are useful for wound closure application. In another embodiment the volume of the cyanoacrylate composition may be increased relatively by a factor of between 2- and 4-fold, for example when the inventive handheld device is used in surgical preparations to provide a microbial barrier application.

### B) Without a non-pressurised compartment

When there is no non-pressurised compartment present in the moulded actuator, means to present the initiator composition of the invention is still required. The initiator composition is then retained within a porous matrix, irrespective of its location, by capillary action or by coating surfaces of pores within an open cell structure of a porous matrix.

In one embodiment the moulded actuator has within its structure only one conduit preferably of circular cross-section and of diameter comprised between 0.5 mm and 3 mm, one end of which terminates either at a dispensing nozzle as described above or at a disc formed from a porous matrix or material. The single conduit is L-shaped having a second end that engages with the pressurized canister via a valve. The disc of porous material is retained firmly in place, for example by an annular retaining ring. A removable cap initially protects the disc of porous material. In alternative embodiments of this version of the moulded actuator, a porous matrix may be integrated to the outside of a dispensing nozzle to which the conduit terminates, or an external hollow tube containing a porous material within may be connected to the dispensing nozzle.

It is understood that a porous material entraining initiator composition may also be presented as a plug within an external tube, for example that may be fitted on outside of the dispensing nozzle as an alternative embodiment.

The plastics used in any moulded part are medical grade and inert such as ultra-high molecular weight polyethylene (UHMWPE) or polypropylene and are resistant to sterilization and compatible with CA, alcohols, aqueous solutions, and hexadimethylsyloxane. If in direct contact with CA, the plastics should be preferably unpigmented as is common practice in the art and in commercial high density polypropylene bottles for example, of consumer superglues. Overmoulding may be used to combine plastics of different types in the moulded actuator to achieve colour or other features such as haptic feel if need be.

In a preferred embodiment, the moulded actuator comprises a porous matrix.

The porous matrix or material is of an open cell structure with pore sizes comprised between 40 µm and 300 µm, more preferably between 60 µm and 125 µm, and may be constructed of various materials such as high-density polyolefin, or may be a frit made from glass, ceramic or metals. Such materials are available for instance from Porex Technologies Corp.

The purpose of the porous matrix is to enable activation of the cyanoacrylate composition by comprising an initiator composition entrained within the matrix, which activates the cyanoacrylate composition as it is propelled through its structure.

Preferably the porous matrix is made of ultra-high molecular weight polyethylene.

### Outer housing

The outer housing for the handheld device provides the structure for the device delivering the inventive compositions and encases the pressurized canister and provides shoulders to enable engagement of the actuator and cap. It is moulded from medical grade plastic, preferably PMMA, and it may be transparent or opaque and preferably the latter. The device is of a small hand-held type and should comfortably fit into and average sized hand and may be contoured or not. The outer housing typically bears a location for the product description, name, Brand and any instructions relating to use, contents or safety.

### Pressurised canister

The pressurized canisters for use in the present invention are usually small and economically viable for use in compact devices intended to trade in the same market space as conventional pen-like TSAs that are Class II medical devices and that are mostly used for one or two applications from less than 2 mL of adhesive composition for wound closure application or less than 5 mL for microbial barrier application.

Preferred pressurized canisters are of the valve-released type designed for medical devices and available from type Picocyl LLC, CO 80401 USA, as disclosed in US-A-2017/0258583A1, in customizable fill form. These cylinders have dimensions of approximately 29 mm x 9 mm (1 ml) or 48 mm x 14 mm (4.5 ml).

Either so-called 'cold filling' or 'pressure filling' may be used to fill pressurised canisters. The inventive compositions are either a cyanoacrylate composition or an initiator composition.

Either may consist of a solution or suspension of their functional components(s) dissolved in a solvent or suspended in a carrier that is a liquid at room temperature.

In cold filling, the bulk propellant is placed into a pre-chilled mixing vessel at a temperature low enough to ensure that the propellant is also in liquid form. The composition is added to the cold propellant in the mixing vessel, and the entire formulation is mixed to ensure homogeneity. The chilled-liquid compositions are then filled into the pressurized canisters. The heavy vapours of the cold-liquid propellant will generally displace the air present within the canister. A valve is placed on top of each canister and welded into place, forming a seal between the top of the canister and a gasket within its valve. Each completed canister is checked for weight to ensure the correct amount of composition is in the canister. Compositions in the pressurized canisters remain liquid.

In pressure filling, compositions and propellants or propellant mixtures may be filled under pressure as solutions or suspensions through the valves of pre-assembled sealed canisters that are held open during the filling process. Air oxygen may be left within the canister initially. The pressure in the pressurized canisters is preferably comprised between 2 bar and 10 bar at 25 °C.

### Method

Also disclosed is a method for activating a cyanoacrylate adhesive composition, which comprises the steps of:
a) pressing the moulded actuator to release the component comprised in the pressurized canister of the device of the invention,
b) directing the released component from the pressurised canister into contact with a second component comprised within the actuator of the device and released therefrom so that a mixing of released components occurs, and
c) directing a mixture of released components from the dispensing nozzle of the device at a substrate that is a living organ.

### Use of the device

Also disclosed is the use of the handheld device of the invention for applying an activated cyanoacrylate adhesive composition onto an area of a living organ.

In an embodiment, the use is for applying an activated cyanoacrylate adhesive composition onto a wound site of a living organ to seal the wound.

In an embodiment, the use is for applying an activated cyanoacrylate adhesive composition onto an area of a living organ prior to a surgical incision as microbial barrier.

Whereas one use of the device is to apply an activated cyanoacrylate to a wound site to form a protective film and promote healing, another use is to apply an activated cyanoacrylate to form a film that is a microbial barrier on an area of a living organ within which one or more surgical incisions or punctures are to be purposely made, for example, by a scalpel, needle or cannula, in a surgical operation. In such use the cyanoacrylate composition may comprise additional antimicrobial agents and colourants.

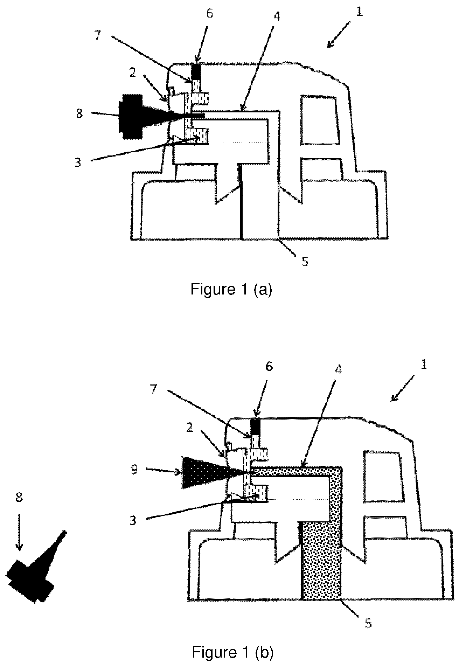

Figure 1 (a) schematically represents a moulded actuator (1) for use in the first embodiment of the invention that comprises a dispensing nozzle having a central orifice (2) that encloses a non-pressurised compartment (3). In this embodiment, the initiator composition is contained that either wholly or partially fills said compartment. The compartment is accessed by a filling port (7), which is subsequently tightly sealed with a stopper (6). Protruding into the compartment is a conduit (4) of L-shape, one end of which terminates close to but not touching the dispensing nozzle (2), and the other end (5) of which engages with a valve in a pressurised canister (not shown). Before its first use, a removable plug or pin (8) prevents the initiator composition (3) entering the conduit (4) or exiting the dispensing nozzle (2).

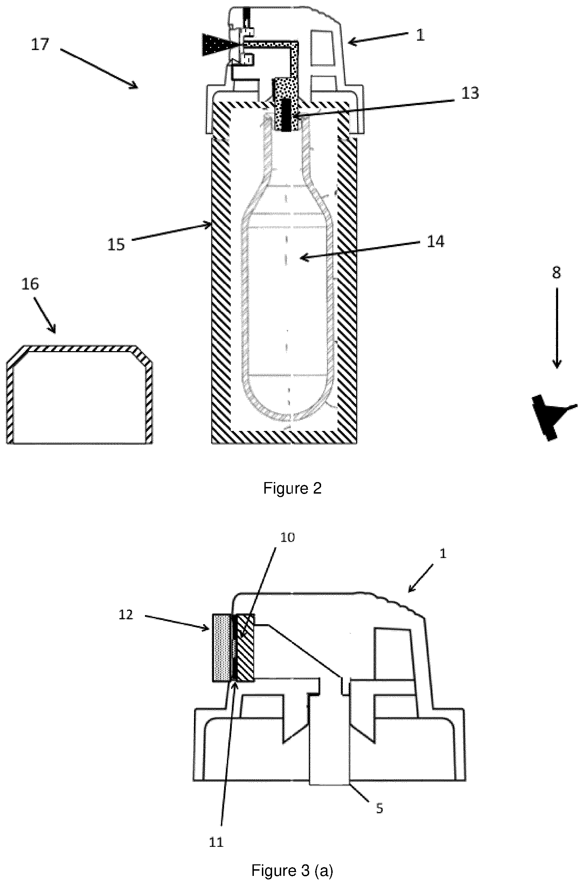

Figure 1 (b) schematically illustrates the status of the moulded actuator (1) in use following removal of the plug or pin (8) illustrating an activated spray (9) being produced following propulsion of what is the cyanoacrylate monomer composition in the present embodiment from a pressurised canister (not shown - see Figure 2) from conduit end (5) through conduit (4) and passing through and aspirating the initiator composition (3).

Due to the nature of the application of the invention only small liquid volumes (2mL or less) are relevant. In the alternative third embodiment, the cyanoacrylate composition is present in non-pressurised compartment (3) and the activated spray (9) is produced following propulsion of the initiator composition from a pressurised canister (not shown - see Figure 2) from conduit end (5) through conduit (4) and passing through and aspirating the cyanoacrylate composition (3).

Figure 2 schematically represents a handheld device (17) for spraying an activated cyanoacrylate adhesive composition deploying an actuator (1) of the type described in Figures 1 (a) and 1 (b). The protective cover, cap or lid (16) of the device is removed, as is the sealing plug or pin (8). A pressurised canister (14) contains either the cyanoacrylate monomer composition or the initiator composition in alternative first and second embodiments respectively and is encased in the outer housing (15). The actuator communicates with the pressurised canister via a spring-loaded valve (13). In use, the end user, removes the cap (16), removes the pin (8) and depresses the actuator (1) causing a confluence of the two compositions by aspirating one into the other thereby invoking activation simultaneously while spraying and while in an ambient pressure environment. The activated spray is directed at the wound site from a suitable distance to cover the injury and without contacting the skin with the device. The polymerising cyanoacrylate quickly forms a protective coating on the wound with a cooling effect due to evaporation of propellant and solvent if present. As with conventional TSAs, the device is designed for one or two shots and then discarded. The materials of the outer housing and pressurised canister can be easily separated and recycled. Specific pressurised canisters could be refilled with specific compositions for new devices that would be re-sterilised.

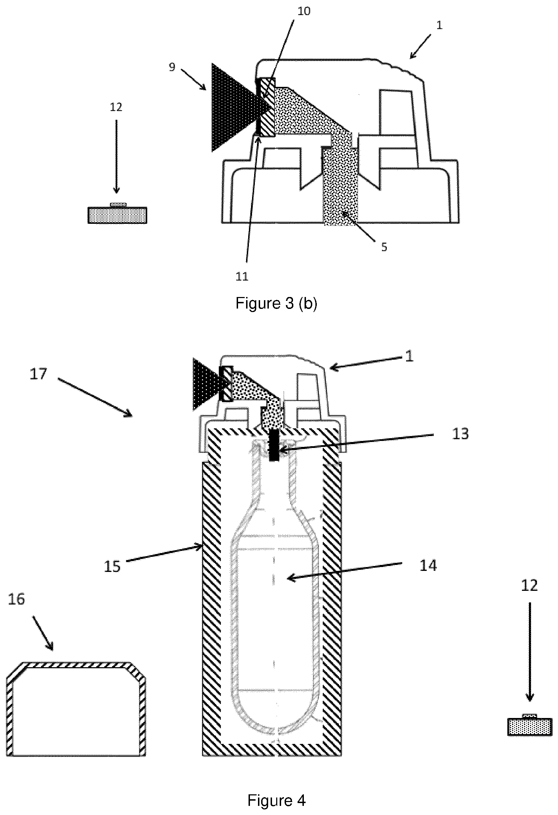

Figure 3 (a) schematically represents a moulded actuator (1) for use in the second embodiment of the invention. In this embodiment there is no non-pressurised compartment within the actuator and a porous matrix (10) terminates the singular L-shaped conduit (5) at one end, while its other end engages with a valve in a pressurised canister (not shown). The porous matrix is in the form of a disc and has an open pore structure. It is firmly held in place by an annular retaining ring (11). Initially before use, the porous matrix disc is protected by a removable cover (12). An initiator composition is entrained within the porous matrix. Figure 3b) schematically illustrates the status of the moulded actuator (1) in use following removal of the cover (12) illustrating an activated spray (9) being produced following propulsion of the cyanoacrylate composition from a pressurised canister (not shown - see Figure 4) from conduit end (5) passing through porous matrix (10) containing the initiator composition.

Figure 4 schematically represents a handheld device (17) for spraying an activated cyanoacrylate composition deploying an actuator (1) of the type described for use in the second embodiment of the invention. The protective cover, cap or lid (16) of the device is removed, as is the protective cover (12) for the porous disc. A pressurised canister (14) contains the cyanoacrylate composition and is encased in the outer housing (15). The actuator communicates with the pressurised canister via a spring-loaded valve (13). In use, the end user, removes the cap (16), removes the protective cover (12) and depresses the actuator (1) causing propulsion of the cyanoacrylate through an open cell porous matrix entraining the initiator composition, thereby invoking activation simultaneously while spraying and while in an ambient pressure environment. The activated spray is directed at the wound site from a suitable distance to cover the injury and without contacting the skin with the device. The polymerising cyanoacrylate quickly forms a protective coating on the wound with a cooling effect due to evaporation of propellant and solvent if present. As with conventional TSAs, the device is designed for one or two shots and then discarded. The materials of the outer housing and pressurised canister can be easily separated and recycled. Specific pressurised canisters could be refilled with specific compositions for new devices that would be re-sterilised.

### Examples

The following methods were used to assess the performance of the CA compositions of the invention.

### 1) Viscosity

The following parameters were used by measuring the viscosity:
- Brookfield DV2T cone/plate viscometer
- Spindle C51Z
- Measuring time: 5 min
- T: 25 °C
- Rotational speed: 0.1 - 200 rpm
- Sample: 1 mL

### 2) Exotherm

The exotherm resulting from the polymerisation of the adhesive composition on a thin polyethylene film substrate was measured with the aid of an infrared sensor placed just below a polyethylene film and the maximum temperature was recorded.

### 3) Wound closure strength

It was measured according to ASTM F2458.

### 4) Peeling resistance in "T"

It was measured according to ASTM F2256.

### 5) Shear strength

It was measured according to ASTM F2255.

### 6) Water penetration test

It was measured according to EN 13726-3.

### 7) Water vapour transmission rate test

It was measured according to ASTM E96.

### Comparative Example 1: Spraying a CA composition without initiator

Distilled n-butyl cyanoacrylate (BCA) stabilised by 1000 ppm butylated hydroxyanisole, 10 ppm sulfur dioxide, 5 ppm boron trifluoride diethyl etherate, and dyed with 50 ppm of D&C violet #2, was placed in a non-pressurised HDPE canister attached to a separate pressurised canister containing a mixture of dimethylether, isobutane and propane used as propellant. A spray nozzle was attached to the pressurised canister that functioned as an actuator allowing, aspiration of the cyanoacrylate formulation whilst simultaneously admixing it with the propellant mixture to form the spray stream.

The initial viscosity of the cyanoacrylate formulation was measured at 25 °C by means of a viscosimeter Brookfield DV2T (spindle C51Z at specific rotational speeds) before spraying.

The spray was also collected after dispensing on a HDPE surface and condensed back to a bulk liquid and its final viscosity again measured in the same way and at the same temperature.

The measurements were recorded as shown in Table I:

**TABLE I**

| Rotational speed (rpm) | Initial viscosity (cP) | Final viscosity (cP) |
|---|---|---|
| 100 | 3.83 | 4.08 |
| 200 | 3.59 | 3.86 |

The experiment showed no activation or polymerisation occurred either induced by admixing n-butyl cyanoacrylate with the propellant mixture alone, or by the act of spraying.

### Comparative Example 2: Spraying a CA composition without initiator

Distilled 2-octyl cyanoacrylate (OCA) stabilised by 2000 ppm butylated hydroxytoluene, 10 ppm sulfur dioxide, and dyed with 50 ppm of D&C violet #2, was placed in a non-pressurised HDPE canister attached to a separate pressurised canister containing a mixture of dimethylether, isobutane and propane used as propellant. A spray nozzle was attached to the pressurised canister that functioned as an actuator allowing, aspiration of the cyanoacrylate formulation whilst simultaneously admixing it with the propellant mixture to form the spray stream.

The initial viscosity of the cyanoacrylate formulation was measured at 25 °C by means of a viscosimeter Brookfield DV2T (spindle C51Z at specific rotational speeds) before spraying.

The spray was also collected after dispensing on a HDPE surface and condensed back to a bulk liquid and its final viscosity again measured in the same way and at the same temperature.

The measurements were recorded as shown in Table II:

**TABLE II**

| Rotational speed (rpm) | Initial viscosity (cP) | Final viscosity (cP) |
|---|---|---|
| 100 | 5.97 | 5.15 |
| 200 | 5.85 | 5.15 |

The experiment showed no activation or polymerisation occurred either induced by admixing 2-octyl cyanoacrylate with the propellant mixture alone, or by the act of spraying.

### Comparative Example 3: Spraying thickened CA compositions without initiator

Distilled n-butyl cyanoacrylate (BCA) stabilised by 1000 ppm butylated hydroxyanisole, 10 ppm sulfur dioxide, 5 ppm boron trifluoride diethyl etherate, and dyed with 50 ppm of D&C violet #2, was formulated with various thickeners to obtained higher viscosity cyanoacrylate formulations. The formulations were placed in a non-pressurised HDPE canister attached to a separate pressurised canister containing a mixture of dimethylether, isobutane and propane used as propellant. A spray nozzle was attached to the pressurised canister that functioned as an actuator allowing, aspiration of the cyanoacrylate formulation whilst simultaneously admixing it with the propellant mixture to form the spray stream.

The initial viscosity of the various thickened cyanoacrylate formulations was measured at 25 °C by means of a viscosimeter Brookfield DV2T (spindle C51Z at specific rotational speeds) before spraying. The spray was also collected after dispensing on a HDPE surface and condensed back to a bulk liquid and its final viscosity again measured in the same way and at the same temperature. The measurements were recorded as a function of type and concentration of thickener as shown in Table III:

**TABLE III**

| Thickener | % | Rotational speed (rpm) | Initial viscosity (cP) | Final viscosity (cP) |
|---|---|---|---|---|
| PMMA* | 10 | 4 | 1183.0 | 1204.3 |
| VA-VC copolymer** | 10 | 10 | 318.9 | 296.1 |
| Hydrophobic fumed silica | 3 | 10 | 209.5 | 213.7 |

| | | | | |
|---|---|---|---|---|
| * Poly(methyl methacrylate) ** Vinyl acetate-vinyl chloride copolymer | | | | |

The experiments showed no activation or polymerisation occurred either induced by admixing thickened n-butyl cyanoacrylate with the propellant mixture alone, or by the act of spraying. Relatively viscous formulations could be sprayed readily.

### Comparative Example 4: Spraying a sterilized tissue CA composition without initiator

A sterilized tissue adhesive formulation (e-beam - 25 kGy), comprised of distilled n-hexyl cyanoacrylate (HCA) monomer 85 % (w/w) stabilised by 2000 ppm butylated hydroxyanisole and 200 ppm sulfur dioxide, D&C violet #2 50 ppm, glycerol triacetate 6 % (w/w), vinyl acetate-vinyl chloride copolymer 7 % (w/w), and hydrophobic fumed silica 2 % (w/w), was placed in a non-pressurised HDPE canister attached to a separate pressurised canister containing a mixture of dimethylether, isobutane and propane used as propellant. A spray nozzle was attached to the pressurised canister that functioned as an actuator allowing, aspiration of the cyanoacrylate formulation whilst simultaneously admixing it with the propellant mixture to form the spray stream.

The initial viscosity of the cyanoacrylate formulation was measured at 25 °C by means of a viscosimeter Brookfield DV2T (spindle C51Z at specific rotational speeds) before spraying. The spray was also collected after dispensing on a HDPE surface and condensed back to a bulk liquid and its final viscosity again measured in the same way and at the same temperature.

The measurements were recorded as shown in Table IV:

**TABLE IV**

| Rotational speed (rpm) | Initial viscosity (cP) | Final viscosity (cP) |
|---|---|---|
| 0.1 | 3350 | 2427 |
| 1 | 538.8 | 553.4 |
| 5 | 260.2 | 274.8 |
| 10 | 207.3 | 212.1 |

The experiment showed no activation or polymerisation occurred either induced by admixing a sterile n-butyl cyanoacrylate with the propellant mixture alone, or by the act of spraying.

### Comparative Example 5: Mixing CA compositions with initiator

Distilled n-butyl cyanoacrylate (BCA) stabilised by 1000 ppm butylated hydroxyanisole, 10 ppm sulfur dioxide and 5 ppm boron trifluoride diethyl etherate, was mixed in a vial with an initiator composition comprised of a quaternary ammonium salt or a tertiary amine in a polar solvent to promote polymerization. The resulting polymers were dissolved in THF in a ratio of 2 mg/ml to determine their molecular weight by Gel Permeation Chromatography (GPC) using an Agilent 1260 Infinity instrument calibrated with poly(methyl methacrylate) standards. The molecular weights attainable as a function of initiator type and concentration in specific solvents are shown in Table V:

**TABLE V**

| Solvent | Initiator | Concentration (ppm) | Mw (g/mol) |
|---|---|---|---|
| Water | Benzalkonium chloride | 937 | 4.49 x 10⁵ |
| | | 2811 | 4.94 x 10⁵ |
| | Choline chloride | 831 | 4.50 x 10⁵ |
| | | 2495 | 5.53 x 10⁵ |
| | Triethanolamine | 907 | 4.24 x 10⁵ |
| | | 2722 | 3.89 x 10⁵ |
| Ethanol | Benzalkonium chloride | 900 | 6.44 x 10⁴ |
| | | 2700 | 2.60 x 10⁴ |
| | Choline chloride | 890 | 4.21 x 10⁴ |
| | | 2670 | 2.83 x 10⁴ |
| | Triethanolamine | 989 | 1.32 x 10⁵ |
| | | 2967 | 1.35 x 10⁵ |

The experiment illustrated that polymers of significant molecular weight resulted in a convenient manner initiated by small volumes of initiator solutions of low concentration.

### Comparative Example 6: Mixing CA compositions with initiator

Distilled *n*-butyl cyanoacrylate (BCA) stabilised by 1000 ppm butylated hydroxyanisole, 10 ppm sulfur dioxide and 5 ppm boron trifluoride diethyl etherate, was mixed in a vial with an initiator composition comprised of a quaternary ammonium salt or a tertiary amine in a polar solvent to promote polymerization. Without delay the mixture was applied in bulk liquid form atop a very thin polyethylene film substrate and formed a cured polycyanoacrylate film of approximately 50 mm x 50 mm x 0.5-1 mm dimensions.

The exotherm resulting from the polymerisation was measured with the aid of an infrared sensor placed just below the polyethylene film and the maximum temperature was recorded. A maximum polymerization temperature of 40 °C is considered the threshold for discomfort and/or pain associated with temperature experienced by live skin. Tissue damage is believed to occur over 45 °C. The maximum temperature was recorded as a function of initiator type and concentration in various initiator solutions as indicated in Table VI:

**TABLE VI**

| Solvent | Initiator | Concentration (ppm) | Maximum temperature (°C) |
|---|---|---|---|
| Water | Benzalkonium chloride | 937 | 29.45 |
| | | 2811 | 30.23 |
| | Choline chloride | 831 | 25.69 |
| | | 2495 | 26.6 |
| | Triethanolamine | 907 | 66.64 |
| | | 2722 | 48.25 |
| Ethanol | Benzalkonium chloride | 900 | 66.32 |
| | | 2700 | 57.04 |
| | Choline chloride | 890 | 31.19 |
| | | 2670 | 37.98 |
| | Triethanolamine | 989 | 29.45 |
| | | 2967 | 30.23 |
| Triacetin | 2-(morpholinothio) benzothiazole | 1667 | 42.43 |
| | | 5000 | 34.47 |
| | | 10000 | 45.81 |

The experiment illustrated that the maximum temperature of the exotherm resulting from the polymerisation depends both on the solvent and on the initiator type and concentration.

### Example 1: Spraying a CA composition with initiator

Distilled *n*-butyl cyanoacrylate (BCA) stabilised by 1000 ppm butylated hydroxyanisole, 10 ppm sulfur dioxide and 5 ppm boron trifluoride diethyl etherate, was placed in one part of a pressurised Teflon canister, while an initiator composition comprised of a quaternary ammonium salt or a tertiary amine in a solvent, such as, for example, triacetin, was placed in a non-pressurised separate compartment of the Teflon canister. By depressing an actuator of the pressurised canister, the stabilised cyanoacrylate composition within was released and simultaneously co-mixed with the initiator composition delivered from the non-pressurised compartment of the canister to form an activated spray stream.

In contrast to Comparative Example 1 (see below), the spray stream condensed onto a substrate to immediately form a solid polymerised film. The resulting polymer was dissolved in THF in a ratio of 2 mg/ml to analyse its molecular weight by Gel Permeation Chromatography (GPC) in a calibrated Agilent 1260 Infinity instrument. The molecular weight of the polymeric film as a function of the initiator concentration (5000 ppm or 10000 ppm) was close to 1 million mass units, i.e., 7.21 x 10⁵ g/mol - 7.36 x 10⁵ g mol.

### Example 2: Spraying a CA composition with initiator

Distilled *n*-butyl cyanoacrylate (BCA) stabilised by 1000 ppm butylated hydroxyanisole, 10 ppm sulfur dioxide and 5 ppm boron trifluoride diethyl etherate, was placed in one part of a pressurised Teflon canister, while an initiator composition comprised of a quaternary ammonium salt or a tertiary amine in a polar solvent was placed in a non-pressurised separate part of the Teflon canister.

The mixture was applied in spray form atop a very thin polyethylene film substrate and formed a cured polycyanoacrylate film of approximately 50 mm x 50 mm x 0.5-1 mm dimensions.

The exotherm resulting from the polymerisation was measured with the aid of an infrared sensor placed just below the polyethylene film and the maximum temperature was recorded.

The maximum temperature of a CA composition comprising 10000 ppm of 2-(morpholinothio)benzothiazole as initiator and triacetin as solvent was 34.5 °C (standard deviation 3.8), which is suitable to be applied on live skin.

### Example 3: Spraying a sterilized tissue CA adhesive composition with initiator

A sterilized tissue adhesive formulation (e-beam - 25 kGy), comprised of distilled n-butyl cyanoacrylate monomer 80 % (w/w) stabilised by 2000 ppm butylated hydroxyanisole and 200 ppm sulfur dioxide, D&C violet #2 50 ppm, glycerol triacetate 12 % (w/w), vinyl acetate-vinyl chloride copolymer 6 % (w/w), and hydrophobic fumed silica 2 % (w/w) was placed in a pressurised stainless-steel canister filled with isobutane as propellant.

Then the mixture was propelled through a porous steel matrix comprising choline chloride as absorbed initiator with the aid of an actuator and a commercial spray nozzle. The thus activated formulation was sprayed over pig skin specimens to be cojoined and prepared according to standard methods for tensile testing and various additional tests, such as, for example, disclosed in US-A-2011/0117047A1.

The results and descriptions are summarised in Table VII together with those obtained from a commercial tissue adhesive (Dermabond Advanced^{®}) applied and used in bulk liquid form.

Tensile testing of glued specimens was performed on a calibrated Instron 1100 tensiometer.

**TABLE VII**

| Test | Standard Test Method | Dermabond Advanced^{®} | Example 3 |
|---|---|---|---|
| Wound Closure Strength (Mpa) | ASTM F2458 | 0.04 | 0.06 |
| Peeling resistance in "T" (Mpa) | ASTM F2256 | 0.18 | 0.34 |
| Shear Strength (Mpa) | ASTM F2255 | 0.31 | 0.37 |
| Water Penetration Test | EN 13726-3 | No penetration | No penetration |
| Water Vapour Transmission Rate Test (g/m²/24 h) | ASTM E96 | 158.30 | 130.80 |

It was observed that the tissue adhesive composition applied as a spray stream according to one of the embodiments of the present invention shows comparable performance to commercially available tissue adhesives applied in bulk.

### Example 4: Spraying a sterilized tissue CA adhesive composition with initiator

A sterilized tissue adhesive formulation (e-beam - 25 kGy), comprised of distilled n-hexyl cyanoacrylate (HCA) monomer 85 % (w/w) stabilised by 2000 ppm butylated hydroxyanisole, and 200 ppm sulfur dioxide, D&C violet #2 50 ppm, glycerol triacetate 6 % (w/w), vinyl acetate-vinyl chloride copolymer 7 % (w/w), and hydrophobic fumed silica 2 % (w/w), was placed in a pressurised stainless-steel canister filled with isobutane as propellant.

Then the mixture was propelled through a porous steel matrix comprising choline chloride as an absorbed initiator with the aid of an actuator and a commercial spray nozzle. The thus activated formulation was sprayed over pig skin specimens to be cojoined and prepared according to standard methods for tensile testing and various additional tests, as exposed above.

The results and descriptions are summarised in Table VIII together with those obtained from a commercial tissue adhesive (Dermabond Advanced^{®}) applied and used in bulk liquid form.

Tensile testing of glued specimens was performed on a calibrated Instron 1100 tensiometer.

**TABLE VIII**

| Test | Standard Test Method | Dermabond Advanced^{®} | Example 4 |
|---|---|---|---|
| Wound Closure Strength (Mpa) | ASTM F2458 | 0.04 | 0.06 |
| Peeling resistance in "T" (Mpa) | ASTM F2256 | 0.18 | 0.21 |
| Shear Strength (Mpa) | ASTM F2255 | 0.31 | 0.35 |
| Water Penetration Test | EN 13726-3 | No penetration | No penetration |
| Water Vapour Transmission Rate Test (g/m²/24 h) | ASTM E96 | 158.30 | 205.45 |

It was observed that the tissue adhesive composition applied as a spray stream according to one of the embodiments of the present invention shows comparable performance to commercially available tissue adhesives applied in bulk.

### Example 5: Spraying a sterilized tissue CA adhesive composition with initiator

A sterilized tissue adhesive formulation (e-beam - 25 kGy), comprised of distilled 2-octyl cyanoacrylate (OCA) monomer 90 % (w/w) stabilised by 2000 ppm butylated hydroxytoluene, and 100 ppm sulfur dioxide, D&C violet #2 50 ppm, and polyvinyl acetate 10 % (w/w), was placed in a pressurised stainless-steel canister filled with isobutane as propellant.

Then the mixture was propelled through a porous steel matrix comprising choline chloride as an absorbed initiator with the aid of an actuator and a commercial spray nozzle. The thus activated formulation was sprayed over pig skin specimens to be cojoined and prepared according to standard methods for tensile testing and various additional tests.

The results and descriptions are summarised in Table IX together with those obtained from a commercial tissue adhesive (Dermabond Advanced TM) applied and used in bulk liquid form. Tensile testing of glued specimens was performed on a calibrated Instron 1100 tensiometer.

**TABLE IX**

| Test | Standard Test Method | Dermabond Advanced^{®} | Example 5 |
|---|---|---|---|
| Wound Closure Strength (Mpa) | ASTM F2458 | 0.04 | 0.04 |
| Peeling resistance in "T" (Mpa) | ASTM F2256 | 0.18 | 0.21 |
| Shear Strength (Mpa) | ASTM F2255 | 0.31 | 0.31 |
| Water Penetration Test | EN 13726-3 | No penetration | No penetration |
| Water Vapour Transmission Rate Test (g/m²/24 h) | ASTM E96 | 158.30 | 172.91 |

It was observed that the tissue adhesive composition applied as a spray stream according to one of the embodiments of the present invention shows comparable performance to commercially available tissue adhesives applied in bulk.

### Example 6: Spraying a sterilized tissue CA adhesive composition with initiator

A sterilized tissue adhesive formulation (e-beam - 25 kGy), comprised of distilled 2-octyl cyanoacrylate (OCA) monomer 90 % (w/w) stabilised by 2000 ppm butylated hydroxytoluene, and 100 ppm sulfur dioxide, D&C violet #2 50 ppm, and polyvinyl acetate 10 % (w/w), was placed in a pressurised stainless-steel canister filled with isobutane as propellant.

Then the mixture was propelled through a porous steel matrix comprising choline chloride as an absorbed initiator with the aid of an actuator and a commercial spray nozzle. The thus activated formulation was sprayed over a large sample of pig skin. When cured an incision was made through the cured polymer film and through the underlying pig skin that was protected by the polymer film.

It was observed that the tissue adhesive composition applied as a spray stream according to one of the embodiments of the present invention is functional as CA based surgical drape.

## Claims

1. A handheld device (17) for applying a cyanoacrylate adhesive composition, **characterized in that** it comprises:
a) a pressurized canister (14) comprising a cyanoacrylate composition comprising a cyanoacrylate monomer, and an initiator composition comprised in a non-pressurized compartment (3),
wherein the device is configured such that the cyanoacrylate composition can be propelled from the pressurized cannister (14) and
the initiator composition can be aspirated from the non-pressurized compartment (3);
wherein the confluence of the cyanoacrylate composition and of the initiator composition occurs in a common activated spray stream (9), and
wherein the pressurized canister (14) comprises a propellant.

2. A handheld device (17) for applying a cyanoacrylate adhesive composition, that it comprises:
b) a pressurized canister (14) comprising a cyanoacrylate composition comprising a cyanoacrylate monomer, and a porous matrix (10) comprising an initiator composition entrained in the porous matrix (10),
wherein the device is configured such that the cyanoacrylate composition can be propelled through the porous matrix (10) comprising
an initiator composition entrained therein,
wherein the confluence of the cyanoacrylate composition and of the initiator composition occurs in a common activated spray stream (9), and **characterized in that** the pressurized canister (14) comprises a propellant.

3. A handheld device (17) for applying a cyanoacrylate adhesive composition, **characterized in that** it comprises:
c) a pressurized canister (14) comprising an initiator composition and a non-pressurized compartment (3) comprising a cyanoacrylate composition comprising a cyanoacrylate monomer,
wherein the device is configured such that the initiator composition can be propelled from the pressurized cannister (14) and the cyanoacrylate composition can be aspirated from the non-pressurized compartment (3),
wherein the confluence of the cyanoacrylate composition and of the initiator composition occurs in a common activated spray stream (9), and
wherein the pressurized canister (14) comprises a propellant.

4. The device according to any one of claims 1 to 3, **characterized in that** the cyanoacrylate composition comprises a cyanoacrylate monomer and a further component selected from radical polymerization inhibitors, acid stabilizing agents, thickening agent, plasticizers, thixotropic agents, accelerating agents, solvents, dyestuffs, and mixtures thereof.

5. The device according to any one of claims 1 to 4, **characterized in that** the cyanoacrylate monomer is selected from the group of n-butyl cyanoacrylate, n-hexyl cyanoacrylate, 2-octyl cyanoacrylate, and mixtures thereof.

6. The device according to any one of claims 1 to 5, **characterized in that** the propellant is selected from chlorofluorocarbons, hydrocarbons, hydrochlorofluorocarbons, hydrofluorocarbons, nitrogen, nitrous oxide (N₂O), carbon dioxide, and mixtures thereof.

7. The device according to any one of claims 1 to 6, **characterized in that** either the cyanoacrylate or the initiator compositions, or both, include at least one antimicrobial agent.

8. The device according to any one of claims 1 to 7, **characterized in that** it comprises a protective cap (16), a moulded actuator (1), an outer housing (15), and a pressurized canister (14) comprising an actuable valve (13).

9. The device according to claim 8, **characterized in that** the moulded actuator (1) comprises a porous matrix (10).

10. The device according to claim 9, **characterized in that** the porous matrix (10) comprises an initiator composition entrained within.

11. The device according to any one of claims 1 to 10, **characterized in that** the pressure in the pressurized canisters (14) is comprised between 2 bar and 10 bar.

12. The device according to any one of claims 1 to 11 suitable for use for applying an activated cyanoacrylate adhesive composition onto an area of a living organ.

13. The device according to claim 12 suitable for applying an activated cyanoacrylate adhesive composition onto a wound site of a living organ to seal the wound.

14. The device according to claim 12 suitable for applying an activated cyanoacrylate adhesive composition onto an area of a living organ prior to a surgical incision as microbial barrier.

## Patentansprüche

1. Handgerät (17) zum Auftragen einer Cyanacrylat-Klebstoffzusammensetzung, **dadurch gekennzeichnet, dass** es Folgendes umfasst:
a) einen unter Druck stehenden Kanister (14), der eine Cyanacrylatzusammensetzung, die ein Cyanacrylat-Monomer umfasst, und eine Initiatorzusammensetzung, die in einer nicht unter Druck stehenden Kammer (3) enthalten ist, enthält,
wobei das Gerät so konfiguriert ist, dass die Cyanacrylatzusammensetzung aus dem unter Druck stehenden Kanister (14) ausgestoßen werden kann und die Initiatorzusammensetzung aus der nicht unter Druck stehenden Kammer (3) angesaugt werden kann; wobei das Zusammenfließen der Cyanacrylatzusammensetzung und der Initiatorzusammensetzung in einem gemeinsamen aktivierten Sprühstrom (9) erfolgt, und
wobei der unter Druck stehende Kanister (14) ein Treibmittel umfasst.

2. Handgerät (17) zum Auftragen einer Cyanacrylat-Klebstoffzusammensetzung, **dadurch gekennzeichnet, dass** es Folgendes umfasst:
b) einen unter Druck stehenden Kanister (14), der eine Cyanacrylatzusammensetzung, die ein Cyanacrylat-Monomer umfasst, und eine poröse Matrix (10), die eine in der porösen Matrix (10) eingeschlossene Initiatorzusammensetzung umfasst, umfasst,
wobei das Gerät so konfiguriert ist, dass die Cyanacrylatzusammensetzung durch die poröse Matrix (10) getrieben werden kann, die eine darin eingeschlossene Initiatorzusammensetzung umfasst,
wobei das Zusammenfließen der Cyanacrylatzusammensetzung und der Initiatorzusammensetzung in einem gemeinsamen aktivierten Sprühstrom (9) erfolgt, und **dadurch gekennzeichnet, dass** der unter Druck stehende Kanister (14) ein Treibmittel umfasst.

3. Handgerät (17) zum Auftragen einer Cyanacrylat-Klebstoffzusammensetzung, **dadurch gekennzeichnet, dass** es Folgendes umfasst:
c) einen unter Druck stehenden Kanister (14), der eine Initiatorzusammensetzung umfasst, und eine nicht unter Druck stehende Kammer (3), die eine Cyanacrylatzusammensetzung umfasst, die ein Cyanacrylat-Monomer umfasst, wobei das Gerät so konfiguriert ist, dass die Initiatorzusammensetzung aus dem unter Druck stehenden Kanister (14) getrieben werden kann und die Cyanacrylatzusammensetzung aus der nicht unter Druck stehenden Kammer (3) angesaugt werden kann, wobei das Zusammenfließen der Cyanacrylatzusammensetzung und der Initiatorzusammensetzung in einem gemeinsamen aktivierten Sprühstrom (9) erfolgt, und
wobei der unter Druck stehende Kanister (14) ein Treibmittel umfasst.

4. Gerät nach einem der Ansprüche 1 bis 3, **dadurch gekennzeichnet, dass** die Cyanacrylatzusammensetzung ein Cyanacrylat-Monomer und eine weitere Komponente, ausgewählt aus Radikalpolymerisationsinhibitoren, Säurestabilisatoren, Verdickungsmitteln, Weichmachern, Thixotropiermitteln, Beschleunigern, Lösungsmitteln, Farbstoffen und Mischungen davon, umfasst.

5. Gerät nach einem der Ansprüche 1 bis 4, **dadurch gekennzeichnet, dass** das Cyanacrylat-Monomer ausgewählt ist aus der Gruppe von n-Butylcyanacrylat, n-Hexylcyanacrylat, 2-Octylcyanacrylat und Mischungen davon.

6. Gerät nach einem der Ansprüche 1 bis 5, **dadurch gekennzeichnet, dass** das Treibmittel ausgewählt ist aus Chlorfluorkohlenwasserstoffen, Kohlenwasserstoffen, Chlorwasserstofffluorkohlenwasserstoffen, Fluorkohlenwasserstoffen, Stickstoff, Distickstoffmonoxid (N₂O), Kohlendioxid und Mischungen davon.

7. Gerät nach einem der Ansprüche 1 bis 6, **dadurch gekennzeichnet, dass** entweder die Cyanacrylat- oder die Initiatorzusammensetzung oder beide mindestens ein antimikrobielles Mittel enthalten.

8. Gerät nach einem der Ansprüche 1 bis 7, **dadurch gekennzeichnet, dass** es eine Schutzkappe (16), einen geformten Aktor (1), ein Außengehäuse (15) und einen unter Druck stehenden Kanister (14) mit einem betätigbaren Ventil (13) umfasst.

9. Gerät nach Anspruch 8, **dadurch gekennzeichnet, dass** der geformte Aktor (1) eine poröse Matrix (10) umfasst.

10. Gerät nach Anspruch 9, **dadurch gekennzeichnet, dass** die poröse Matrix (10) eine darin eingeschlossene Initiatorzusammensetzung umfasst.

11. Gerät nach einem der Ansprüche 1 bis 10, **dadurch gekennzeichnet, dass** der Druck in den unter Druck stehenden Kanistern (14) zwischen 2 bar und 10 bar liegt.

12. Gerät nach einem der Ansprüche 1 bis 11, geeignet zum Auftragen einer aktivierten Cyanacrylat-Klebstoffzusammensetzung auf einen Bereich eines lebenden Organs.

13. Gerät nach Anspruch 12, geeignet zum Auftragen einer aktivierten Cyanacrylat-Klebstoffzusammensetzung auf eine Wundstelle eines lebenden Organs zum Verschließen der Wunde.

14. Gerät nach Anspruch 12, geeignet zum Auftragen einer aktivierten Cyanacrylat-Klebstoffzusammensetzung auf einen Bereich eines lebenden Organs vor einem chirurgischen Schnitt als mikrobielle Barriere.

## Revendications

1. Un dispositif portatif (17) pour appliquer une composition adhésive à base de cyanoacrylate, **caractérisé en ce qu'**il comprend :
a) une boîte pressurisée (14) comprenant une composition de cyanoacrylate comprenant un monomère de cyanoacrylate, et une composition d'initiateur comprise dans un compartiment non pressurisé (3),
dans lequel le dispositif est configuré de manière à ce que la composition de cyanoacrylate puisse être propulsée à partir de la boîte pressurisée (14) et que la composition d'initiateur puisse être aspirée à partir du compartiment non pressurisé (3) ; dans lequel la confluence de la composition de cyanoacrylate et de la composition d'initiateur se produit dans un flux de pulvérisation activé commun (9), et
dans lequel la boîte pressurisée (14) comprend un agent propulseur.

2. Un dispositif portatif (17) pour appliquer une composition adhésive à base de cyanoacrylate, comprenant :
b) une boîte pressurisée (14) comprenant une composition de cyanoacrylate comprenant un monomère de cyanoacrylate, et une matrice poreuse (10) comprenant une composition d'initiateur entraînée dans la matrice poreuse (10), dans lequel le dispositif est configuré de manière à ce que la composition de cyanoacrylate puisse être propulsée à travers la matrice poreuse (10) comprenant une composition d'initiateurs entraînée dans celle-ci,
dans lequel la confluence de la composition de cyanoacrylate et de la composition d'initiateur se produit dans un flux de pulvérisation activé commun (9), et **caractérisé par le fait que** la boîte pressurisée (14) comprend un agent propulseur.

3. Un dispositif portatif (17) pour appliquer une composition adhésive à base de cyanoacrylate, **caractérisé en ce qu'**il comprend :
c) une boîte pressurisée (14) comprenant une composition d'initiateur et un compartiment non pressurisé (3) comprenant une composition de cyanoacrylate comprenant un monomère de cyanoacrylate,
dans lequel le dispositif est configuré de manière à ce que la composition d'initiateur puisse être propulsée à partir de la boîte pressurisée (14) et que la composition de cyanoacrylate puisse être aspirée à partir du compartiment non pressurisé (3), dans lequel la confluence de la composition de cyanoacrylate et de la composition d'initiateur se produit dans un flux de pulvérisation activé commun (9), et
dans lequel la boîte pressurisée (14) comprend un agent propulseur.

4. Dispositif de contrôle selon l'une quelconque des revendications 1 à 3, **caractérisé par le fait que** la composition de cyanoacrylate comprend un monomère de cyanoacrylate et un autre composant choisi parmi les inhibiteurs de polymérisation radicalaire, les agents stabilisateurs d'acide, les agents épaississants, les agents plastifiants, les agents thixotropes, les agents accélérateurs, les solvants, les colorants et leurs mélanges.

5. Dispositif de contrôle selon l'une quelconque des revendications 1 à 4, **caractérisé par le fait que** le monomère cyanoacrylate est choisi dans le groupe du cyanoacrylate de n-butyle, le cyanoacrylate de n-hexyle, le cyanoacrylate de 2-octyle et leurs mélanges.

6. Dispositif de contrôle selon l'une quelconque des revendications 1 à 5, **caractérisé par le fait que** l'agent propulseur est choisi parmi les chlorofluorocarbones, les hydrocarbures, les hydrochlorofluorocarbures, les hydrofluorocarbures, l'azote, l'oxyde nitreux (N₂O), le dioxyde de carbone et leurs mélanges.

7. Dispositif de contrôle selon l'une quelconque des revendications 1 à 6, **caractérisé par le fait que** les compositions de cyanoacrylate ou d'initiateur, ou les deux, comprennent au moins un agent antimicrobien.

8. Dispositif de contrôle selon l'une quelconque des revendications 1 à 7, **caractérisé par le fait qu'**il comprend un capuchon de protection (16), un actionneur moulé (1), un boîtier extérieur (15), et un réservoir pressurisé (14) comprenant une valve actionnable (13).

9. Dispositif selon la revendication 8, **caractérisé par le fait que** l'actionneur moulé (1) comprend une matrice poreuse (10).

10. Dispositif selon la revendication 9, **caractérisé par le fait que** la matrice poreuse (10) comprend une composition d'initiateur entraînée à l'intérieur.

11. Dispositif de contrôle selon l'une quelconque des revendications 1 à 10, **caractérisé par le fait que** la pression dans les bidons pressurisés (14) est comprise entre 2 bars et 10 bars.

12. Le dispositif selon l'une des revendications 1 à 11 peut être utilisé pour appliquer une composition adhésive à base de cyanoacrylate activé sur une zone d'un organe vivant.

13. Dispositif selon la revendication 12, adapté à l'application d'une composition adhésive à base de cyanoacrylate activé sur le site d'une plaie d'un organe vivant pour sceller la plaie.

14. Dispositif selon la revendication 12, adapté à l'application d'une composition adhésive à base de cyanoacrylate activé sur une zone d'un organe vivant avant une incision chirurgicale, en tant que barrière microbienne.
